# EUROPEAN PATENT APPLICATION

(11) **EP 1 764 090 A1**
(43) Date of publication of application: **21.03.2007**
(21) Application number: 05020217.5
(22) Date of filing: 15.09.2005
(51) Int. Cl.: A61K 9/127

(54) **Amphoteric liposomes for local drug applications**

(71) Applicant: novosom AG, 06120 Halle (DE)
(72) Inventor: Panzner, Steffen. Dr., 06108 Halle (DE); Panzner, Cornelia, 06108 Halle (DE); Lutz, Silke. Dr., 06114 Halle (DE); Endert, Gerold. Dr., 06114 Halle (DE)
(74) Representative: Ziebig, Marlene

(57) **Abstract**

To provide pharmaceutical compositions for a local application of nucleic acid therapeutics to mucous membranes, ex vivo to grafts before transplantation and to the eyes and in the area of inflammatory or immune-mediated diseases and disorders pharmaceutical compositions, comprising at least a) amphoteric liposomes having an isoelectric point between 4 and 7 and b) one or more nucleic acid therapeutics and c) a pharmaceutically acceptable carrier wherein the pH is 3-5, more preferably 4-5 are proposed.

## Description

### Field of the invention

Nucleic acid therapeutics represent a new class of drugs for which both systemic and local applications have been tested and are currently under development. Excluding CpG-oligos or aptamers, the majority of these compounds has an intracellular site of action. Carrier systems are used to overcome the poor uptake of such substances and are sometimes an indispensable prerequisite.
Amphoteric liposomes represent a recently described class of liposomes having an anionic or neutral charge at pH7.5 and a cationic charge at pH4. These liposomes are very well tolerated in animals and can encapsulate nucleic acid molecules with high efficiency. The invention provides selected amphoteric liposomes being stable to pH changes and pharmaceutical compositions comprising such amphoteric liposomes. The use of such products is advantageous in the local application of nucleic acid based therapeutics to mucous membranes, ex vivo to grafts before transplantation and to the eyes. Such pharmaceutical compositions are useful in the prevention or treatment of various diseases as well as for genetic vaccination.

### Definitions

Within that disclosure the following definitions should apply:
**Amphoter or amphoteric character:** A structure, being a substance or a mixture of substances or a supramolecular complex (e.g. a liposome) comprising charged groups of both anionic and cationic character wherein
   (i) at least one of the charged groups has a pK between 4 and 8 and
   (ii) the cationic charge prevails at pH 4 and
   (iii) the anionic charge prevails at pH 8,
      resulting in an isoelectric point of neutral net charge between pH4 and pH8. Amphoteric character by that definition is different from zwitterionic character, as zwitterions do not have a pK in the range mentioned above. In consequence, zwitterions are essentially neutrally charged over a range of pH values. Phosphatidylcholine or phosphatidylethanolamins are neutral lipids with zwitterionic character.
**Active drug** within the invention means one or more nucleic acid based therapeutics
**Excipient** within that invention means a liposome comprising one or more lipids, in particular an amphoteric liposome, but not comprising active drug or a specific vehicle.

**Vehicle** within that invention means a pharmaceutically acceptable carrier comprising water, buffer substances, salts, sugars, polymers and the like but not comprising active drug or excipient.

**Formulation** within that invention means the combination of nucleic and excipient without specifying the vehicle

**Composition** within that invention means the combination of active drug, excipient and vehicle.

### Background of the invention

Nucleic acid therapeutics are classified into nucleic acids that are encoding for one or more specific sequences for proteins, polypeptides, or RNAs and in oligonucleotides that can very specifically downregulate protein expression.

Oligonucleotides comprise antisense, locked nucleic acids (LNA), peptide nucleic acids (PNA), morpholino nucleic acids (Morpholinos), small interfering RNAs (siRNA) or decoys of various chemistries. A detailed description of the different mechanism can be found in the literature (eg. Crooke in BBA (1999), 1489(1), 31-44; Tijsterman et al. in Cell (2004), 117(1), 1-3 or Mann et al. in J Clin Invest, (2000), 106(9), 1071-5.

It is known in the art, that nucleic acid therapeutics, irrespective of their actual chemical origin lack therapeutic efficacy due to instability in body fluids or inefficient uptake into cells or for both reasons. Chemical modifications of the oligonucleotide comprising but not limited to the abovementioned variants, also extended towards the formation of conjugates with ligands or polymers represent one strategy to overcome practical limitations. A second set of strategies involves the use of carrier systems, in particular liposomes for protection, targeting and enhanced uptake into cells. Such carrier system shall meet an optimum score of the following criteria high encapsulation efficiency and economic production process, colloidal stability of the carrier, enhanced uptake into cells and of course low toxicity and immunogenicity.

Anionic or neutral liposomes are often excellent in terms of colloidal stability, as no aggregation occurs between the carrier and the environment. Consequently their biodistribution is excellent and the potential for irritation and cytotoxicity is low. However, such carrier lack encapsulation efficiency and do not provide an endosomolytic signal that facilitates further uptake into cells (Journal of Pharmacology and experimental Therapeutics (2000), 292, 480-488 by Klimuk et al.).

A great many of publications deal with cationic liposomal systems, e.g. Molecular Membrane Biology (1999), 16, 129-140 by Maurer et al.; BBA (2000) 1464, 251-261 by Meidan et al. ; Reviews in Biology and Biotechnology (2001), 1(2), 27-33 by Fiset & Gounni . Although cationic systems provide high loading efficiencies, they lack colloidal stability, in particular after contact with body fluids. Ionic interactions with proteins and/or other biopolymers lead to in situ aggregate formation with the extracellular matrix or with cell surfaces. Cationic lipids have often been found to be toxic as shown by Filion et al. in BBA (1997), 1329(2), 345-358; Dass in J. Pharm. Pharmacol. (2002), 54(5), 593-601; Hirko et al. in Curr. Med. Chem., 10(14), 1185-1193.

These limitations were overcome by the addition of components that provide a steric stabilization to the carriers. Polyethylenglycols of various chain length are known to eliminate the aggregation problems associated with the use of cationic components in body fluids and PEGylated cationic liposomes show enhanced circulation times in vivo (BBA (2001) 1510, 152-186 by Semple et al.). Still, the use of PEG does not solve the intrinsic toxicity problem associated with the cationic lipids. It is also known that PEG substantially inhibits the productive entry of such liposomes into the cells or their intracellular delivery (Song et al. in BBA (2002), 1558(1), 1-13).

Amphoteric liposomes represent a recently described class of liposomes having an anionic or neutral charge at pH7.5 and a cationic charge at pH4. Explicit reference is made here to WO 02/066490, WO 02/066012 and the WO 03/070735, all to Panzner et al. which give a detailed description of the amphoteric liposomes. Further disclosures are made in WO 03/070220 and WO 03 070735, also to Panzner et al. describing more pH sensitive lipids for the manufacturing of said amphoteric liposomes. Amphoteric liposomes have an excellent biodistribution and are very well tolerated in animals. They can encapsulate nucleic acid molecules with high efficiency.

Nucleic acid therapeutics represent a new class of drugs for the treatment of a variety of diseases. In addition to a systemic application there are a lot of preclinical and clinical studies especially in the area of inflammatory or immune-mediated diseases and disorders and in the field of genetic vaccination that deal with local applications of the drugs to mucous membranes, ex vivo to grafts and to the eyes (eg. Shanahan in Expert Opin Investig Drugs, (1999), 8(9), 1417-1429; Ball et al. in Am J Pharmacogenomics, (2003), 3(2), 97-106; Finotto et al. in J Allergy Clin Immunol., (2002), 107(2), 279-286; Nedbal et al. in Antisense Nucleic Acid Drug Dev., (2002), 12(2), 71-78; Bochot et al. in Prog Retin Eye Res., (2000), 19(2), 131-147; Rogy et al. in Human Gene Therapy, (2000), 11(12), 1731-1741; Klavinskis in J. Immunol, (1999), 162, 254-262; Hopson et al. in Methods (2003), 31(3), 217-224; Barnes et al. in Curr Opin Mol Ther. (2000), 2(1), 87-93.

### Object of the invention

Therefore, the object of the invention is to provide stable formulations of amphoteric liposomes for a local application of nucleic acid therapeutics to mucous membranes, ex vivo to grafts before transplantation and to the eyes.

Another object of the invention is the local application of the pharmaceutical compositions in the area of inflammatory or immune-mediated diseases and disorders.

### Summary of the invention

The present invention is directed to pharmaceutical compositions comprising amphoteric liposomes and nucleic acid therapeutics, which can be locally administered to mucous membranes, and to the eyes and ex vivo to grafts. The amphoteric liposomes are negatively or neutrally charged at pH 7.4 and cationic at pH 4. A substantial portion or all of said nucleic acid therapeutics is physically entrapped into the amphoteric liposomes. The invention also relates to preferred types of amphoteric liposomes that are stable at a slightly acidic pH. Together with suitable and preferred vehicles, pharmaceutical compositions are described that can be administered at this pH. In a preferred embodiment of the invention, the formulations can be lyophilized The pharmaceutical compositions of the present invention further can be used for other topical treatments of a condition and/or disease in mammals or of parts of mammals, in particular humans or their organs.
In a preferred embodiment of the invention the pharmaceutical compositions can be used for the topical treatment of inflammations, immune or autoimmune disorders and for genetic vaccination.

### Detailed description of the invention

It was found that specific amphoteric liposomes are stable both at pH 7,5 as well as at pH 4-5 and that a local application of antisense loaded amphoteric liposomes at pH 4-5 is in particular effective in the treatment of inflammatory diseases or immune-related disorders.
It was also found, that nucleic acid loaded amphoteric liposomes can be lyophilized at pH 4-5. Thus, amphoteric liposomes are offering means for both providing a stable storage form as well as facilitating effective drug application.

Amphoteric liposomes as described before are negatively or neutrally charged at pH 7.4 and cationic at pH 4. To improve the bioadhesion of amphoteric liposomes to mucous membranes after a local application it is advantageous to have a cationic surface charge of the liposomes. Amphoteric liposomes are cationic at a slightly acidic pH, more precisely at a pH below the isoelectric point of the respective liposome. The application of the amphoteric liposomes at this pH requires that the amphoteric liposomes do not aggregate or fuse under such conditions. The aggregation or fusion of amphoteric liposomes at an acidic pH depends on the lipid composition of the amphoteric liposomes and the presence of cargo. It was found that specific empty and drug loaded amphoteric liposomes are stable upon a pH-shift to 4-5.

For example, amphoteric liposomes built up of the charged lipids OOTAP and Chems are only stable at an acidic pH when the neutral lipid POPC is also present in the bilayer. Stable means that the liposomes do not aggregate upon acidification. In contrast, the exchange of POPC with DOPE leads again to a destabilization of the membrane at low pH. This destablization was also found for a range of cation:anion ratios in the mixture.
In another example, amphoteric liposomes built from MoChol and CHEMS form stable bilayers with a mixture of POPC and DOPE.

The examples given below give further mixtures of amphoteric liposomes suitable for practicing the invention. As the invention is not limited to the examples, an assay for identifying and testing other amphoteric liposomes is also described.

Amphoteric liposomes of the present invention comprise
a) a neutral phospholipid being either phosphatidylcholine or mixtures of phosphatidylcholine and phosphatidylethanolamine
b) optionally cholesterol
c) an amphoteric lipid or alternatively a mix of lipid components with amphoteric properties.

In a preferred embodiment of the invention neutral phosphatidylcholins or mixtures of phosphatidylcholins and phosphoethanolamins are present in the mixture to at least 20mol%, more preferred to at least 30% and even more preferred to more than 40%. Preferred components are selected from the group of DMPC, DPPC, DSPC, POPC, DOPC or from phosphatidylcholins from natural sources such as soy bean PC or egg PC. Preferred phosphatidylethanolamins are selected from DOPE or DMPE or DPPE. Most preferred neutral lipids are DOPE, POPC, soy bean PC or egg PC.

Amphoteric lipids are disclosed in the WO 02/066489 as well as in the WO 03/070735. More preferably, the amphoteric lipids are selected from the group of HistChol, HistDG, isoHistSuccDG, Acylcarnosin and HCCHol.
Most preferred the amphoteric lipid is HistChol.
Amphoteric liposomes of the present invention may also be manufactured using pH-responsive anionic and/or cationic components, as disclosed in WO 02/066012. Cationic lipids sensitive to pH are disclosed in WO 02/066489 A2/A3 and WO 03/070220 A1 and in the references made therein, in particular in Budker et al. 1996, Nat Biotechnol. 14(6):760-4 and can be used in combination with constitutively charged anionic lipids or with anionic lipids that are sensitive to pH. Vice versa, the cationic charge may also be introduced from constitutively charged lipids that are known to those skilled in the art in combination with a pH sensitive anionic lipid. Combinations of constitutively charged anionic and cationic lipids, e.g. DOTAP and DPPG are not preferred.
Preferred cationic components are selected from the group of DPIM, CHIM, DORIE, DDAB, DAC-Chol, TC-Chol, DOTMA, DOGS, (C18)₂Gly⁺ N,N-dioctadecylamido-glycin, CTAB, CPyC, DODAP and DOEPC.

Further preferred cationic lipids are DMTAP, DPTAP, DOTAP, DC-Chol, MoChol and HisChol.
The amphoteric mixtures further comprise anionic lipids, either constitutively or conditionally charged in response to pH and those lipids are also known to those skilled in the art. Preferred lipids for use with the invention are selected from the group comprising: DOGSucc, POGSucc, DMGSucc, DPGSucc, DMPS, DPPS, DOPS, POPS, DMPG, DPPG, DOPG, POPG, DMPA, DPPA, DOPA, POPA, CHEMS and CetylP.
Further preferred anionic lipids are DOGSucc, DMGSucc, DMPG, DPPG, DOPG, POPG, DMPA, DPPA, DOPA, POPA, CHEMS and CetylP

Abbreviations for lipids refer primarily to standard use in the literature and are included here as a helpful reference:
- DMPC: Dimyristoylphosphatidylcholin
- DPPC: Dipalmitoylphosphatidylcholin
- DSPC: Distearoylphosphatidylcholin
- POPC: Palmitoyl-oleoylphosphatidylcholin
- DOPC: Dioleoylphosphatidylcholin
- DOPG: Dioleoylphosphatidylglycerol
- POPG: Palmitoyl-oleoylphosphatidylglycerol
- DMPG: Dimyristoylphosphatidylglycerol
- DPPG: Dipalmitoylphosphatidylglycerol
- DMPS: Dimyristoylphosphatidylserin
- DPPS: Dipalmitoylphosphatidylserin
- DOPS: Dioleoylphosphatidylserin
- POPS: Palmitoyl-oleoylphosphatidylserin
- DMPA: Dimyristoylphosphatidic acid
- DPPA: Dipalmitoylphosphatidic acid
- DOPA: Dioleoylphosphatidic acid
- POPA: Palmitoyl-oleoylphosphatidic acid
- Chems: Cholesterolhemisuccinat
- DC-Chol: 3-β-[N-(N',N-dimethylethane) carbamoyl]cholesterol
- CetylP: Cetylphosphat
- DODAP: (1,2)-dioleoyloxypropyl)-N,N-dimethylammonium chloride
- DOEPC: 1,2-dioleoyl-sn-glycero-3-ethylphosphocholin
- DAC-Chol: 3-β-[N-(N,N'-dimethylethane) carbamoyl]cholesterol

- TC-Chol: 3-13-[N-(N',N', N'-trimethylaminoethane) carbamoyl] cholesterol
- DOTMA: (1,2-dioleyloxypropyl)-N,N,N-trimethylammoniumchlorid) (Lipofectin®)
- DOGS: ((C18)₂GlySper3") N,N-dioctadecylamido-glycyl-spermin (Transfectam®)
- CTAB: Cetyl-trimethylammoniumbromid,
- CPyC: Cetyl-pyridiniumchlorid
- DOTAP: (1,2-dioleoyloxypropyl)-N,N,N-trimethylammonium Salz
- DMTAP: (1,2-dimyristoyloxypropyl)-N,N,N-trimethylammonium Salz
- DPTAP: (1,2-dipalmitoyloxypropyl)-N,N,N-trimethylammonium Salz
- DOTMA: (1,2-dioleyloxypropyl)-N,N,N-trimethyfammonium chlorid)
- DORIE: (1,2-dioleyloxypropyl)-3 dimethylhydroxyethyl ammoniumbromid)
- DDAB: Dimethyldioctadecylammonium bromid
- DPIM: 4-(2,3-bis-palmitoyloxy-propyl)-1-methyl-1H-imidazole
- CHIM: Cholesterol-(3-imidazol-1-yl propyl)carbamate
- MoChol: 4-(2-Aminoethyl)-Morpholino-Cholesterolhemisuccinat
- HisChol: Histaminyl-Cholesterolhemisuccinat.
- HCChol: Nα-Histidinyl-Cholesterolcarbamat
- HistChol: Nα-Histidinyl-Cholesterol-hemisuccinat
- AC: Acylcarnosin, Stearyl- & Palmitoylcarnosin
- HistDG: 1,2-Dipalmitoylglycerol-hemisuccinat-Nα-Histidinyl-hemisuccinat,& Distearoyl- ,Dimyristoyl, Dioleoyl or palmitayl-oleoyltierivatives
- IsoHistSuccDG: 1,2-Dipalmitoylglycerol-Oα-Histidinyl-Nα-hemisuccinat, & Distearoyl-, Dimyristoyl, Dioleoyl or palmitoyl-oleoylderivatives
- DGSucc: 1,2-Dipalmitoyglycerol-3-hemisuccinat & Distearoyl-, dimyristoyl-Dioleoyl or palmitoyl-oleoylderivatives

| | |
|---|---|
| MoChol | DG-Succ |
| 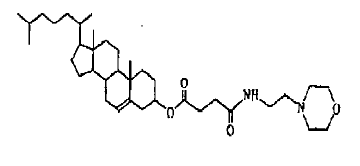 | 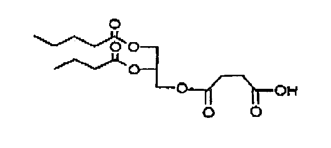 |
| DOTAP | IsohistsucoDG |
| 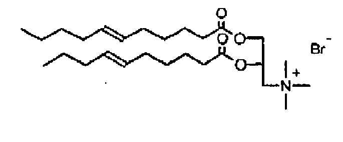 | 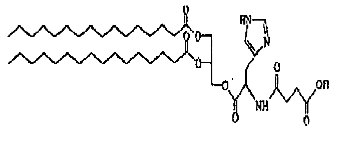 |
| HisChol | HCChol |
| 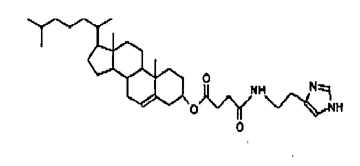 | 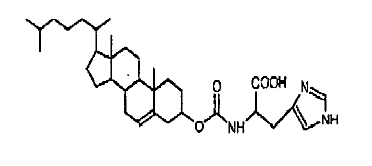 |
| AC | |
| 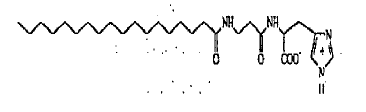 | |
| Hist-Chol | |
| 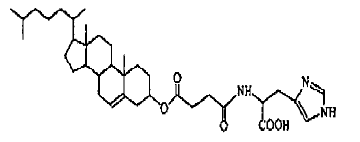 | |
| Hist-DG | |
| 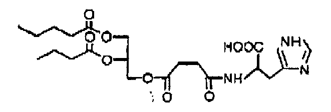 | |

Active drugs of the current invention are nucleic acid based. These are classified into nucleic acids that are encoding for one or more specific sequences for proteins, polypeptides, or RNAs and into oligonucleotides that can very specifically down regulate protein expression.

In one embodiment of the invention, the nucleic acid based therapeutics are encoding for one or more specific sequences for proteins, polypeptides, or RNAs and eventually are translated into such proteins, polypeptides or RNAs. Such nucleic acid therapeutics can be circular DNA plasmids, linear DNA constructs, like MIDGE vectors (Minimalistic Immunogenically Defined Gene Expression) as disclosed in WO9821322A1 and DE19753182A1 or mRNAs ready for translation (eg. EP1392341B1).

In another embodiment of the invention, oligonucleotides are used that can target existing intracellular nucleic acids encoding for a specicific protein thereby modulating the expression of the protein. The term "target nucleic acid" encompass DNA encoding for a specific protein as well as all RNAs derived from such DNA, being pre-mRNA or mRNA. A specific hybridisation between the target nucleic acid and one or more oligonucleotides directed against such sequences result in an inhibition of protein expression. To achieve such specific targeting, the oligonucleotide must comprise a continuous stretch of nucleotides being complementary to the sequence of the target nucleic acid.

Oligonucleotides fulfilling the abovementioned criteria may be built with a number of different chemistries and topologies. Oligonucleotides may be single stranded or double stranded. Single stranded oligonucleotides include but are not limited to DNA-based oligonucleotides, locked nucleic acids, 2'-modified oligonucleotides and others, commonly known as antisense oligonucleotides. Backbone or base modifications may include but are not limited to Phosphothioate DNA (PTO), 2'O-methyl RNA (2'Ome), 2' O- methoxyethyl-RNA (2'MOE), peptide nucleic acids (PNA), N3'-P5' phosphoamidates (NP), 2'fluoroarabino nucleic acids (FANA), locked nucleic acids (LNA), Morpholine phosphoamidate (Morpholino), Cyclohexene nucleic acid (CeNA), tricyclo-DNA (tcDNA) and others. Moreover, mixed chemistries are known in the art, being constructed from more than a single nucleotide species as copolymers, blockcopolymers or gapmers or in other arrangements.

In addition to the aforementioned oligonucleotides, a protein expression can also be inhibited using double stranded RNA molecules containing the complementary sequence motifs. Such RNA molecules are known as siRNA molecules in the art (eg.

WO9932619A1 or WO02055693A2). Again, various chemistries were adapted to this class of oligonucleotides. Also. DNA / RNA hybrid systems are known in the art.

In another embodiment of the present invention, decoy oligonucleotides can be used. These double stranded DNA molecules do not target nucleic acids but transcription factors. This means that decoy oligonucleotides bind sequence-specific DNA-binding proteins and interfere with the transcription (eg. Cho-Chung et al. in Curr Opin Mol Ther., 1999).

All above mentioned oligonucleotides may vary in length between as little as 10, preferably 15 and even more preferably 18 and 50, preferably 30 and more preferably 25 nucleotides. The fit between the oligonucleotide and the target sequence is preferably perfect with each base of the oligonucleotide forming a base pair with its complementary base on the target nucleic acid over a continuous stretch of the abovementioned number of oligonucleotides. The pair of sequences may contain one mismatch within the said continuous stretch of base pairs, although this is less preferred.

Methods for the manufacturing of liposomes are known to those skilled in the art. They include but are not limited to extrusion through membranes of defined pore size, injection of lipid solutions in ethanol into the water phase containing cargo or high pressure homogenization.
Also, it is known in the art that nucleic acid therapeutics can be contacted with the excipient at neutral pH, resulting in volume inclusion of a certain percentage of the solution containing the nucleic acid. High concentrations of excipients ranging from 50mM to 150mM are preferred to achieve substantial encapsulation of the drug.

In contrast to such standard procedure, amphoteric liposomes offer the distinct advantage of binding nucleic acids at or below their isoelectric point and thereby concentrating the drug at the liposome surface. Such process is described in WO 02/066012 A2/A3 in more detail.

Irrespective of the actual production process the non-encapsulated active drug can be removed from the liposomes after the initial production step wherein liposomes are formed as tight containers. Again, the technical literature and the references included here describe such methodology in detail and suitable process steps may include but are not limited to size exclusion chromatography, sedimentation, dialysis, ultrafiltration or diafiltration and the like.
in a preferred embodiment of the invention more than 80°!0 of the drug are inside the liposomes.

However, such removal of non-encapsulated material is not mandatory and in one preferred embodiment of the invention the formulation comprises entrapped as well as free drug.

The particle size of the formulation is between 50 and 500nm, more preferred between 100 and 500nm and even more preferred between 150 and 300nm.

After the manufacturing process lyophilization of the formulation provides further means for stabilization. In one preferred embodiment of the present invention the composition is lyophilized at the abovementioned acidic pH and reconstituted with water for injection prior to use. The acidic pH during lyophilization and subsequent reconstitution prevent loss of encapsulated nucleic acid material due to an interaction of the drugs with the liposomal membrane. If lyophilization is part of the manufacturing procedure, protecting agents such as sugars or amino acids or polymers can be present in the vehicle.

Although the application of the pharmaceutical composition is done with particular advantage at a lower pH, practising the invention is of course not limited to that. In one embodiment of the present invention, the formulations can be applied at a physiological pH between 7 and 8.

In one preferred embodiment of the present invention, the formulations can be applied at a slightly acidic pH, in particular at a pH below the isoelectric point of the respective excipient. More preferred the pH of the composition is not lower than pH 3.5 and most preferred the composition has a pH between 4 and 5 when applied.

Pharmaceutical acceptable vehicles for such application are known to those skilled in the art and include but are not limited to acetic acid, citric acid or glycine and the like for compositions having the desired pH.

As low pH is detrimental to the long-term stability of the used nucleic acids and /or lipids, the pH is preferentially adjusted to the lower value before use. Means to achieve this under pharmacologically acceptable standards are known to those of ordinary skill in the art and include but are not limited to mixing the storage stable colloid with an appropriate amount of acetic acid, citric acid or glycine, preferentially buffered to a lower pH, more preferred buffer between pH2 and pH4.

Some of the following combinations (A-D) of process steps are used with specific advantage. The non-encapsulated drug can be removed from the pharmaceutical composition, but this step is not mandatory.
(A)
   - encapsulation of drug at neutral pH
   - vehicle can be water, saline or buffered saline
   - actual liposome formation and sizing step
   - non- entrapped drug removed
   - storage form suspension .
   - pH is adjusted below the isoelectric point of the respective excipient
   - administration at acidic pH
(B)
   - encapsulation of drug at neutral pH
   - vehicle can be water, saline or buffered saline
   - actual liposome formation and sizing step
   - non- entrapped drug removed
   - pH is adjusted below the isoelectric point of the respective excipient and addition of protectants
   - lyophilization
   - storage form: powder
   - reconstitution and administration at acidic pH
(C)
   - encapsulation of drug at a pH below the isoelectric point of excipient using a molar ratio of cationic charges of the excipient to anionic charges of the drug between 0,5 and 20 and more preferred between 1 and 10
   - vehicle can be buffered with acetic acid, citric acid and the like and may further contain sodium chloride or sucrose.
   - actual liposome formation and sizing step
   - addition of cryoprotectants and lyophilization
   - storage form: powder
   - reconstitution and administration at acidic pH
(D)
   - encapsulation of drug at a pH below the isoelectric point of excipient using a molar ratio of cationic charges of the excipient to anionic charges of the drug between 0,5 and 20 and more preferred between 1 and 10
   - vehicle can be buffered with acetic acid, citric acid and the like and may further contain sodium chloride or sucrose.
   - actual liposome formation and sizing step
   - raise pH to neutrality
   - non-entrapped drug removed
   - select a combination of further process steps from (A), (B), (C).

The present invention describes pharmaceutical composition comprising nucleic acid therapeutics for a local application to mucous membranes, ex vivo to grafts before transplantation and to the eyes. Without being limited to the examples given here, such compositions are therapeutically active in the treatment of inflammatory bowel disease In general the compositions are useful for the prevention or treatment of different conditions or diseases in mammals. One specific task is the local application of the compositions in the prevention or treatment of inflammations, immune or autoimmune disorders, including but not limited to graft rejection, graft-versus-host disease, inflammatory bowel disease, Morbus Crohn, Colitis ulcerosa, Asthma bronchiale, COPD, and the like.

Also, reasonable application of said formulations is believed to be within the skill of those in the art. Dosing is dependent on severity and responsiveness of the disease state treated, with the course of treatment lasting from several days to several month, or until cure is effected or a diminution of the disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of the individual drug in the composition and can generally be estimated based on EC50 values found to be effective in animal models. The dosage may be given daily, weekly, monthly or yearly or even less regular. Persons of ordinary skill in the art can easily estimate repetition rates for dosing based on measured residence times and concentrations of the drug in body fluids or tissues. Following successful treatment, it may be desirable to have the patient undergo maintenance therapy to prevent the recurrence of the disease state, wherein the formulation is administered at maintenance doses, once or more daily to once in a year. While the present invention has been described with specificity in accordance with certain of its preferred embodiments, the following examples serve only to illustrate the invention and are not intended to limit the same.

### Example 1: Preparation of amphoteric liposomes

**Tab. 1:**

| **Lipids** | **Composition** |
|---|---|
| POPC/DOTAP/Chems | 60:10:30 |
| POPC/DOTAP/Chems | 40:15:45 |
| POPC/DOTAP/Chems | 20:20:60 |
| POPC/DOTAP/Chems | 25:75 |

A mixture of lipids was dissolved in chloroform and evaporated in abound bottom flask to dryness under vacuum. Lipid films were hydrated with PBS, pH 7.5. The resulting lipid concentration was 50 mM. The suspensions were hydrated for 25 minutes in a water bath at room temperature, sonicated for 5 minutes and frozen at -70°C. After thawing the liposomal suspensions were extruded 15 times through polycarbonate membranes with a pore size of 200nm.

### Example 2: pH-shift experiment with empty amphoteric liposomes

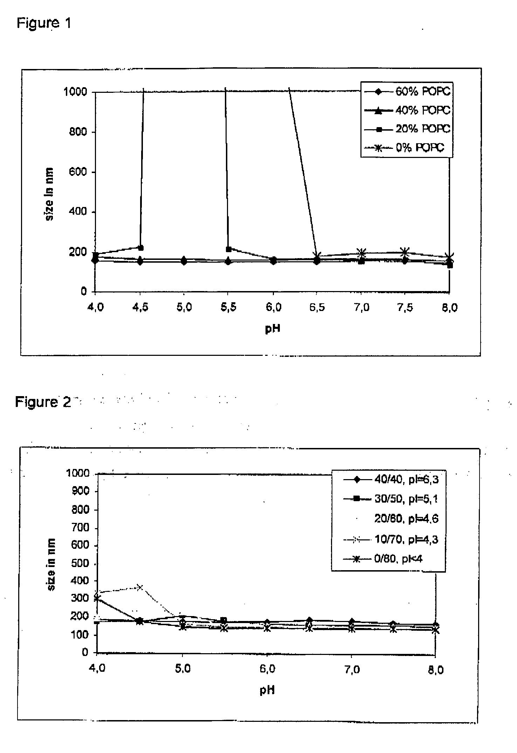

10 µl liposomes of example 1 were diluted 1:100 in 100 mM Citrate/Phosphate-buffer pH 4-8 and incubated for one hour at room temperature. Then 7.5 ml 0,9 % saline was added and the size of the liposomes was characterized by dynamic light scattering. Results are presented in figure 1. Amphoteric liposomes built up of the charged lipids DOTAP and Chems in a ratio 1:3 are only stable at an acidic pH when the neutral lipid POPC is also present in the bilayer with at least 40%.

### Example 3: Preparation of carboxyfluorescein (CF) loaded liposomes

**Tab. 2:**

| **Lipids** | **Composition** |
|---|---|
| POPC/DOTAP/Chems | 20:40:40 |
| POPC/DOTAP/Chems | 20:30:50 |
| POPC/DOTAP/Chems | 20:20:60 |
| POPC/DOTAP/Chems | 20:10:70 |
| POPC/DOTAP/Chems | 20:0:80 |

**Tab. 3:**

| **Lipids** | **Composition** |
|---|---|
| DOPE/DOTAP/Chems | 20:40:40 |
| DOPE/DOTAP/Chems | 20:30:50 |
| DOPE/DOTAP/Chems | 20:20:60 |
| DOPE/DOTAP/Chems | 20:10:70 |
| DOPE/DOTAP/Chems | 20:0:80 |

A mixture of lipids was dissolved in chloroform and evaporated in a round bottom flask to dryness under vacuum. Lipid films were hydrated with 10 µM CF in 10 mM Hepes, 150 mM NaCl, pH 7.5. The resulting lipid concentration was 10 mM. The suspensions were hydrated for 45 minutes in a water bath at room temperature, sonicated for 5 minutes following by three freeze/thaw cycles at -70°C. After thawing the liposomal suspensions were extruded 15 times through polycarbonate membranes with a pore size of 200nm. Non-encapsulated CF was removed by size exclusion chromatography, whereas the liposomes were diluted six fold.

### Example 4: pH-shift experiment with amphoteric liposomes of example 3

A mixture of 150 µl liposomes of example 3, 7.5 ml 0.9 % saline and 150 µl 0,5M Citrate/Phosphate-buffer pH 4-8 was prepared and the size of the liposomes was characterized by dynamic light scattering.

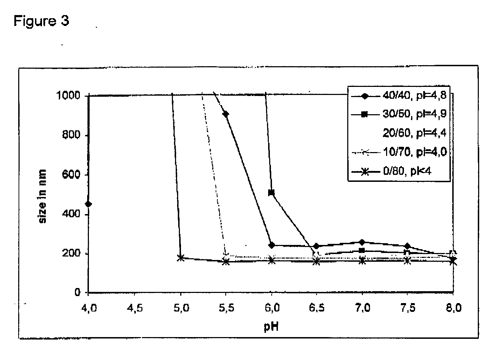

Results are presented in figure 2 and 3. Amphoteric liposomes built up of the charged lipids DOTAP and Chems in different ratios can be stabilized by the presence of POPC but not with DOPE.

### Example 5: Preparation of empty amphoteric liposomes

**Tab. 1**

| **Lipids** | **Composition** |
|---|---|
| POPC/DOPE/MoChol/Chems | 15:45:20:20 |
| POPC/DOTAP/Chems/Chol | 30:10:20:40 |
| POPC/DOTAP/Chems | 60:10:30 |
| POPC/DOTAP/Chems | 60:20:20 |
| POPC/DOPE/MoChot/DMG Succ | 6:24:46:23 |

A mixture of lipids was dissolved in chloroform and evaporated in a round bottom flask to dryness under vacuum. Lipid films were hydrated with PBS, pH 7.5. The resulting lipid concentration was 100 mM. The suspensions were hydrated for 25 minutes in a water bath at room temperature, sonicated for 5 minutes and frozen at -70°C. After thawing the liposomal suspensions were extruded 15 times through polycarbonate membranes with a pore size of 400nm.

### Example 6: Preparation of plasmid-loaded amphoteric liposomes

**Tab. 2**

| **Lipids** | **Composition** | **Plasmid** |
|---|---|---|
| POPC/DOPE/MoChol/Chems | 15:45:20:20 | inside + outside |
| POPC/DOTAP/Chems | 60:10:30 | inside |
| POPC/MoChol/Chems | 30:35:35 | inside |
| | | inside + outside |

Liposomes were produced by injecting 10 Vol-% of an ethanolic lipid solution into 10 mM NaAc 150 mM NaCl pH 4.5 or 10 mM NaAc pH 4.5 containing 16 µg/ml of a 7000 bp plasmid encoding for luciferase. The resulting lipid concentration was 2 mM. The pH of this solution was immediately shifted with 1/10 volume 1M Hepes pH 8. To concentrate the diluted liposomes the suspensions were sedimented for 1h at 80.000 rpm in a TLA 100.4 rotor (Beckman Optima-MAX). To remove non-encapsulated plasmid the concentrated liposomal suspensions were diluted with a sucrose stock solution and brought to 0.8M sucrose. 0.5M sucrose in PBS and pure PBS were layered on top, forming a gradient for removing the plasmid outside of the particles. Sucrose gradients were spun for 45min at 40.000rpm in a MLS-50 rotor (Beckman Optima-MAX) and the liposomes were taken from the upper interphase.

The formulation POPC/DOTAP/Chems 60:10:30 was manufactured by following process:
The lipid mixture was dissolved in chloroform and evaporated in a round bottom flask to dryness under vacuum. Lipid films were hydrated with 10mM NaAc/150 mM NaCl, pH4.5 containing 100 µg/ml plasmid PBS. The resulting lipid concentration was 10 mM. The suspensions were hydrated for 25 minutes in a water bath at room temperature, sonicated for 5 minutes and frozen at -70°C. After thawing the liposomal suspensions were extruded 15 times through polycarbonate membranes with a pore size of 800/200/800 nm. To remove non-encapsulated plasmid the concentrated liposomal suspensions were diluted with a sucrose stock solution and brought to 0.8M sucrose. 0.5M sucrose in PBS and pure PBS were layered on top, forming a gradient for removing the plasmid outside of the particles. Sucrose gradients were spun for 45min at 40.000rpm in a MLS-50 rotor (Beckman Optima-MAX) and the liposomes were taken from the upper interphase.

### Example 7: Stable amphoteric liposomes at pH 4.5

Liposomes were first diluted 1:10 in PBS pH 7.5 and afterwards 1/10 Vol 1M Acetate, pH 4.5 was added very fast. The samples were vortexed immediately after the addition of the shift buffer. Liposomes were characterized by dynamic light scattering.

**Tab. 3: stable amphoteric liposomes after pH-Shift to pH 4.5**

| **Formulation** | **Cargo** | **Size/Pl pH 7.5** | **Size /Pl pH 4.5** |
|---|---|---|---|
| POPC/DOPE/MoChol/Chems 15:45:20:20 | plasmid in + out | 117/0.373 | 266/0.244 |
| | Empty | 193/0.255 | 212/0.195 |
| POPCIDOTAP/Chems/Chol 30:10:20:40 | Empty | 190/0.208 | 202/0.218 |
| POPC/DOTAP/Chems 60:10:30 | plasmid inside | 125/0.091 | 145/0.296 |
| | Empty | 180/0.053 | 179/0.08 |
| POPC/DOTAP/Chems 60:20:20 | Empty | 169/0.138 | 164/0.101 |
| POPC/MoChol/Chems 30:35:35 | plasmid in + out | 109/0.479 | 154/0.240 |
| | plasmid inside | 190/0.177 | 234/0.283 |
| POPC/DOPE/MoChol/DMG Succ 6:24:46:23 | empty | 217/0.113 | 240/0.200 |

### Example 8: Preparation of CD40-ODN-containing liposomes

A mixture of 85 µmol POPC, 42 µmol CHEMS and 14 µmol DOTAP was dissolved in chloroform and evaporated in a round bottom flask to dryness under vacuum.
ODN with the sequence T*C*C*TAGATGGACCGCT*G*T was used with asterisks indicating a phosphorothioate linkage between the nucleotides (after Gao, Ph.D. thesis, Goettingen 2003, rAS3).
Lipid films were hydrated with 1mg ODN in 1mL of buffer (10mM sodium acetate, 150mM NaCl pH4.5). The suspensions were hydrated for 25 minutes in a water bath at room temperature, sonicated for 5 minutes and eventually frozen at -70°C. After thawing the liposomal suspensions were extruded 15 times through polycarbonate membranes with a pore size of 400nm. The liposome suspensions were brought to pH7.5 using 1 M HEPES buffer and to 0.8M sucrose using a stock solution. Nonencapsulated ODN was removed from the extruded sample by flotation through 0.5M sucrose overlaid with 10mM HEPES, 180mM NaCL pH7.5 and the liposome suspension was stored at 4°C. Resulting liposomes were characterized by dynamic light scattering and found to be 220 to 250 nm in size.

### Example 9: Colitis induction

Colitis was induced by using a single intra-colonic application of 2,4,6-trinitrobenzene sulphonc acid (TNBS) prepared by adding 20mg of TNBS to 135µl of 35%ethanol in 150mM NaCl. Male Wistar rats (200...250g) were placed under light ether anaesthesia and the mixture was administered using an 8cm long catheder inserted through the anal canal into the descending colon. After removing the catheder, rats were held in a headfirst position for 30s to avoid flowing out of the enema and rats were kept under normal condition afterwards.

### Example 10: Treatment and analysis

Rats were treated with CD40 antisense from example 1 either 4 hours before or 3 days after the colitis induction. The antisense suspension from example 1 was brought to pH 4.5 using 1 M buffered acetic acid/ sodium acetate pH 4.0 and a total of 100µl containing 2,7µg CD40 antisense suspension was applied to the colon according to example 2.
Seven days after induction of the colitis the animals were sacrificed. The colon was removed and opened longitudinally. Colon samples were fixed in PBS containing 4% formaldehyde. Paraffin-embedded sections (5µm) were stained with haematoxylin/eosin followed by microscopic inspection.

Results are presented in the figures 1 to 3 and demonstrate a very substantial reduction of the experimental colitis when treated with antisense directed against CD40, but not with the scrambled control antisense. Quite surprisingly, even a single treatment of a fully developed colitis at day 3 resulted in a strong and almost complete reduction of the inflammation. In confirmation to that, prevention of the colitis was also achieved when the formulation was applied in a preventive mode before the initiation of the disease.

### Example 11: Alternative Formulation

When used as excipient, a mixture of 60mol% POPC, 20mol% HistChol and 20mol% Cholesterol also resulted in succesful treatment of the experimental colitis.

### Example 12: Non removal of outside Antisense

When used as a formulation, non-removal of non encapsulated antisense also resulted in carrier systems that are stable colloids.

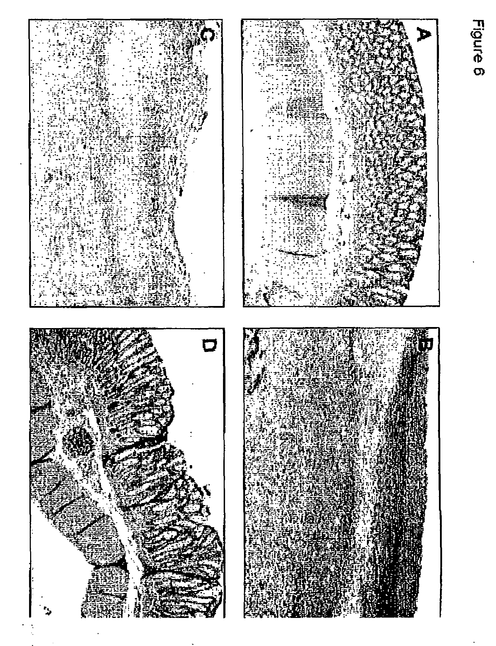

Figure legends
- **Figure 1:**: POPC content was increased within the DOTAP/CHEMS mixture. At least 40% of POPC are needed to completely prevent particle growth at low pH.
- **Figure 2:**: Liposomes were produced at pH 7.5 and adjusted to acidic conditions to promote aggregation. Addition of 20mol% POPC greatly reduces the fusion tendency
- **Figure 3:**: Same as in (Figure 2) but DOPE was tested for stabilization. Particle growth starts at a lower pH when DOTAP/CHEMS 25/75 and DOPEIDOTAP/CHEMS 20/20/60 are compared. Still, all mixtures tested undergo strong aggregation and fusion

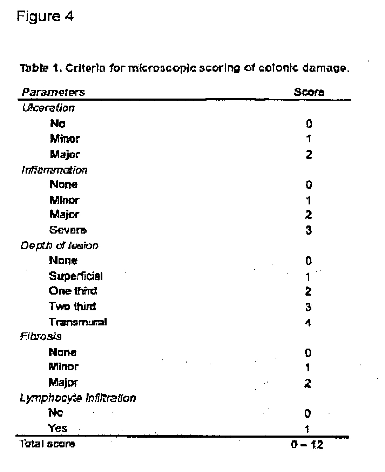

- **Figure 4:**: Criteria for microscopic scoring of colonic damage

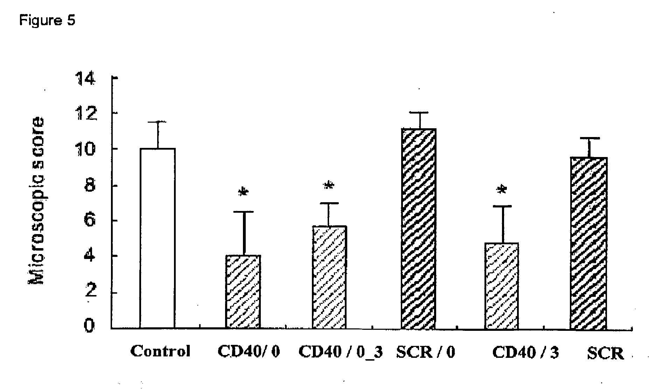

- **Figure 5:**: Microscopic scoring of colonic damage.
Controlcontrol animals, PBS treated
CD40/ 0 treated at day0, 4h prior induction
CD40/ 0_3 treated at day0, 4k prior induction and day3
SCR/ 0treated with scrambled control, 4h prior induction
CD40/ 3 treated at day 3 only
SCR/ 3treated with scrambled control at day 3
- **Figure 6:**: Colon sections after various treatments.
A normal, unaffected bowel wall
B inflamed, but untreated bowel wall
C treatmant prior colitis induction using the scrambled control
D treatment prior colitis induction using the specific CD40 antisense.

## Claims

1. Pharmaceutical compositions, comprising at least a) amphoteric liposomes having an isoelectric point between 4 and 7 and b) one or more nucleic acid therapeutics and c) a pharmaceutically acceptable carrier wherein the pH is 3-5, more preferably 4-5.

2. Pharmaceutical composition of claim 1 wherein the amphoteric liposome comprises amphoteric lipids, neutral phospholipids and optionally cholesterol.

3. Pharmaceutical compositions of claim 2 wherein neutral lipids comprise phosphatidylcholins or mixtures of phosphatidylcholins and phosphoethanolamins and represent at least 20mol%, more preferred at least 30% and even more preferred more than 40% of the total lipids.

4. Pharmaceutical compositions of claim 2 or 3 wherein the neutral lipid is POPC or DOPE or a mixture of both.

5. Pharmaceutical compositions of claim 2 wherein the amphoteric lipid is a single lipid and is selected from the group of HistChol, HistDG, isoHistSuccDG, Acylcarnosin and HCChol.

6. Pharmaceutical compositions of claim 5 wherein the amphoteric lipid is selected from the group of HistChol.

7. Pharmaceutical compositions of claim 2 wherein the amphoteric lipid is a mixture comprising two or more different anionic and cationic lipids selected from the group of DMTAP, DPTAP, DOTAP,DC-Chol, MoChol, HisChol, DPIM, CHIM, DORIE, DDAB,DAC-Chol, TC-Chol, DOTMA, DOGS, (C18)₂Gly N,N-dioctadecylamido-glycin, CTAP, CPyC, DODAP, DOEPC, DGSucc, DMPS, DPPS, DOPS, POPS, DMPG, DPPG, DOPG, POPG, DMPA, DPPA, DOPA, POPA, CHEMS and CetylP.

8. Pharmaceutical compositions of claim 7 wherein the amphoteric lipid mixtures comprise one or more from the group of DOTAP, DC-Chol, MoChol, HisChol, DMGSucc, DOGSucc, DOPA, CHEMS or CetylP.

9. Pharmaceutical compositions of claim 1 wherein the amphoteric liposomes having a size between 50 and 500nm and, more preferentially having a size between 150 and 300nm.

10. Pharmaceutical compositions of one or more of the claims 1 to 9 wherein the nucleic acid encodes for one or more specific sequences for proteins, polypeptides, or RNAs.

11. Pharmaceutical compositions of claim 10 wherein the nucleic acid is a circular DNA plasmid, a linear DNA construct or a mRNA.

12. Pharmaceutical compositions of one or more of the claims 1 to 9 wherein the nucleic acid is a oligonucleotide.

13. Pharmaceutical compositions of claim 12 wherein the oligonucleotide is an antisense oligonucleotide of 15 to 30 basepairs length.

14. Pharmaceutical compositions of claim 12 wherein the oligonucleotide contains phosphothioate linkages.

15. Pharmaceutical compositions of claim 12 wherein the oligonucleotide contains 2'MOE modified nucleobases.

16. Pharmaceutical compositions of claim 12 wherein the oligonucleotide is a siRNA of 15 to 30 basepairs length.

17. Pharmaceutical compositions of claim 12 wherein the oligonucleotide is a decoy oligonucleotide of 15 to 30 basepairs length.

18. Pharmaceutical compositions of one or more of the claims 1 to 17 wherein more than 80 % of the nucleic acid therapeutics are inside the liposomes.

19. Pharmaceutical compositions of one or more of the claims 1 to 18 wherein non-encapsulated nucleic acid therapeutics are not removed from the liposomal suspension.

20. Pharmaceutical compositions of one or more of the claims 1 to 19 wherein the formulations are lyophilized at an acidic pH and reconstituted with water for injection prior to use after the manufacturing process.

21. Use of the pharmaceutical compositions of claim 1 or one of the following for the prevention or treatment of a condition and/or disease in mammals or part of mammals in particular humans or their organs.

22. Method of prevention or treatment of an inflammatory, immune or autoimmune disorder using pharmaceutical compositions of one or more of the claims 1 to 20..

23. Method of treatment for genetic vaccination using pharmaceutical compositions of one or more of the claims 1 to 20.
